# EUROPÄISCHE PATENTSCHRIFT

(11) **EP 0 728 509 B1**
(45) Veröffentlichungstag und Bekanntmachung des Hinweises auf die Patenterteilung: **29.12.1999**
(21) Anmeldenummer: 96102548.3
(22) Anmeldetag: 21.02.1996
(51) Int. Cl.: B01D 19/00, A61M 1/36, A61M 1/00

(54) **Vorrichtung zum Abscheiden von Luftblasen aus medizinischen Flüssigkeiten**
Apparatus for eliminating gas bubbles from medical liquid
Dispositif pour éliminer les bulles de gaz d'un liquide médical

(30) Priorität: 24.02.1995 DE 19506506
(43) Veröffentlichungstag der Anmeldung: 28.08.1996
(73) Patentinhaber: Fresenius Medical Care Deutschland GmbH, 61350 Bad Homburg v.d.H. (DE)
(72) Erfinder: Heilmann, Klaus, D-66606 St. Wendel (DE); Knierbein, Bernd, Dr., D-66606 St. Wendel (DE)
(74) Vertreter: Luderschmidt, Schüler & Partner GbR

(56) Entgegenhaltungen:
- EP-A- 0 284 675
- EP-A- 0 646 380
- US-A- 3 296 779
- US-A- 3 992 172
- US-A- 4 368 118
- US-A- 4 648 890

## Beschreibung

Die Erfindung betrifft eine Vorrichtung zum Abscheiden von Luftblasen aus medizinischen Flüssigkeiten, insbesondere zum Abscheiden von Luftblasen aus Blut, mit einer im wesentlichen kreiszylinderförmigen Kammer, einem Einlaufstutzen und einem Auslaufstutzen.

Da Vorrichtungen zum Abscheiden von Luftblasen aus medizinischen Flüssigkeiten auch zum Abscheiden von anderen Gasen als Luft Verwendung finden können, werden derartige Luftabscheider auch als Entgasungsvorrichtung bezeichnet.

Wenn Blut dem natürlichen Blutkreislauf eines Patienten entnommen und durch einen künstlichen Blutkreislauf geleitet wird, ist es erforderlich, das Blut von eventuell enthaltenen Gasen zu trennen, bevor es wieder dem Körper des Patienten zugeführt wird. Dies kommt beispielsweise bei der Zellseparation im Rahmen der Autotransfusion von Blut bei Operationen vor, weiter bei der Hämodialyse oder Hämofiltration, sowie bei Mischformen dieser Behandlungstechniken.

Insbesondere bei der Entgasung von Blut stellt sich das Problem, daß die Abscheidung von Luftblasen einerseits mit großer Zuverlässigkeit erfolgen muß, da eventuell im Blut enthaltene Luftblasen zum Tode des Patienten führen können, andererseits der Luftabscheider hinsichtlich seiner mechanischen Eigenschaften und der sich ausbildenden Strömungsform so beschaffen sein muß, daß Beschädigungen der Blutbestandteile vermieden werden. Für eine geringe Blutschädigung ist ein gutes Auswaschverhalten des Luftabscheiders wünschenswert, was einhergeht mit materialseitig glatten Oberflächen sowie mit einer strömungsgünstigen kontinuierlichen Gestaltung der Strömungsbahnen, so daß das Anhaften von Blutkörperchen an Oberflächen des Luftabscheiders und damit eine Konglomeration von Blutkörperchen vermieden wird. Für eine geringe Blutschädigung sind weiterhin kurze Verweilzeiten des Blutes im Luftabscheider förderlich, ohne jedoch die Luftabscheidung als solches zu verschlechtern. Ferner ist ein kleines Füllvolumen von Vorteil.

Eine bekannte Vorrichtung zum Abscheiden von Luftblasen aus Flüssigkeiten ist aus GB 2 063 108 bekannt. Der bekannte Luftabscheider weist eine im wesentlichen kreiszylinderförmig ausgebildete, senkrecht angeordnete Kammer mit einem Einlaßstutzen und einem Auslaßstutzen auf. Der Einlaßstutzen ist am oberen Ende der Kammer derart angeordnet, daß die zu entgasende Flüssigkeit im wesentlichen tangential im Bereich des äußeren Umfangs in die Kammer eintritt. Aufgrund der tangentialen Einleitung fließt die zu entgasende Flüssigkeit zunächst auf einer kreisförmigen Strömungsbahn, die jedoch von der gesamten Strömung durch die senkrechte Kammer überlagert wird, so daß die Flüssigkeit die Kammer in einer schraubenlinienförmigen Strömungsbahn durchströmt und am unteren Ende aus dem tangential angeordneten Auslaufstutzen wieder aus der Kammer austritt. Die kreisförmigen Bewegungsanteile der Flüssigkeitsströmung erzeugen dabei Zentrifugalkräfte, die in der Flüssigkeit Druckunterschiede aufbauen, so daß die weniger dichten, d.h. leichtere Luftblasen zur Mitte der Kammer gedrängt werden und längs der Längsachse der Kammer aufsteigen, bis sie durch die Entlüftungsbohrung abgeführt werden.

Die im Krankenhausbetrieb eingesetzten Luftabscheider sollten sich leicht befestigen und ohne größeren Aufwand in bereits bestehende Schlauchleitungen einfügen lassen. Daher wird angestrebt, daß der Einlaßstutzen nicht tangential, sondern in Längsrichtung der Kammer angeordnet ist. Wenn der Einlaßstutzen nämlich auf der Längsachse der Kammer angeordnet ist, laßt sich der Luftabscheider beispielsweise ach Aufschneiden eines existierenden Schlauchs in diesen einsetzen, ohne daß die Schlauchführung geändert werden müßte. Ferner kann die Schlauchführung ohne unnötige Schlaufen erfolgen.

Bei der Verwendung eines Luftabscheiders zur Behandlung von Blut ist es ferner erforderlich, daß der Luftabscheider nach einer gewissen Einsatzzeit und insbesondere bei einem Patientenwechsel ausgetauscht wird. Da eine Reinigung, die den hygienischen Anforderungen im Krankenhausbetrieb genügt, vom Aufwand nicht zu vertreten ist, sollte der Luftabscheider als Wegwerfartikel ausgebildet sein.

Die EP-A-0 646 380, die einen Stand der Technik nach Art. 54(3) EPÜ darstellt, beschreibt einer Luftabscheider der stromab des Einlaufstutzens ein Strömungsleitbauteil aufweist, das im wesentlichen aus einem ratationssymmetrischen Grundkörper besteht, dessen der einströmenden Flüssigkeit zugewandte äußere Oberfläche geometrisch durch Rotation eines Kurvenabschnitts um die Längsachse des Kammer definiert ist und mit Leitschaufeln versehen ist. Parallel zu der ersten Strömungsleitfläche ist eine zweite Leitfläche unter Ausbildung einer Vielzahl von Strömungskanälen angeordnet.

Der Erfindung liegt die Aufgabe zugrunde, eine Vorrichtung zum Abscheiden von Luftblasen aus medizinischen Flüssigkeiten, die einerseits die Vorteile der schraubenlinienförmigen Strömungsführung beibehält und andererseits einen in der Längsachse des Luftabscheiders liegenden Einlaßstutzen aufweist, derart auszubilden, daß deren Herstellung vereinfacht ist.

Die Lösung der Aufgabe erfolgt mit den im Patentanspruch 1 angegebenen Merkmalen.

Bei der erfindungsgemäßen Vorrichtung zum Abscheiden von Luftblasen aus medizinischen Flüssigkeiten weist das stromab des Einlaufstutzens oder Anschlusses angeordnete Strömungsleitbauteil zum Erzeugen der schraubenlinienförmigen Strömungsbahn ein in Längsrichtung der Kammer angeordnetes zentrales Strömungsrohr auf, das in mehrere, vorzugsweise zwei Strömungsleitrohre übergeht, die sich jeweils in einer Raumkurve aus der Kammerlängsrichtung in eine tangential zur Wandung der Kammer verlaufende Richtung erstrecken. Die einströmende Flüssigkeit wird in den Strömungsleitrohren zunächst in radialer Richtung nach außen geleitet, um dann derart umgelenkt zu werden, daß die Flüssigkeit aus den Strömungsleitrohren in einer im wesentlichen tangential zur Wandung der Kammer verlaufenden Richtung ausströmt, wodurch die gewünschte schraubenlinienförmige Strömung induziert wird.

Das Strömungsleitbauteil der erfindungsgemäßen Vorrichtung, das auch als Einlaufverteiler bezeichnet werden kann, läßt sich aufgrund seiner Ausbildung mit den Einströmungsrohren auf einfache Weise als Spritzgußteil aus herkömmlichem, vorzugsweise transparentem Kunststoff in großen Stückzahlen herstellen.

Das Strömungsbauteil ermöglicht es, einen in Längsrichtung der Kammer angeordneten Einlaufstutzen vorzusehen, ohne daß auf die Vorteile des Grundkonzepts, nämlich die Strömung in der Kammer schraubenlinienförmig auszubilden, verzichtet werden muß. Das zentrale Leitrohr des Strömungsleitbauteils kann dabei an dem Einlaufstutzen der Kammer angeschlossen sein, z.B. in den rohrförmigen Einlaßstutzen passend eingesetzt sein. Es ist aber auch möglich, daß das zentrale Strömungsrohr einstückiger Bestandteil des Einlaufstutzens ist.

Mit den Strömungsleitrohren ermöglicht das Strömungsleitbauteil eine kontinuierliche, Stöße weitgehend vermeidende Strömungsführung, so daß in der zu entgasenden Flüssigkeit ein gleichmäßiges Strömungsprofil erzeugt wird. Dies führt zu niedrigen und gleichmäßigen Schubspannungen, so daß das Abscheiden von Luftblasen aus Blut mit einer minimalen Blutschädigung verbunden ist.

Das Strömungsleitbauteil weist vorzugsweise zwei Strömungsleitrohre auf, die derart angeordnet sind, daß deren Öffnungen in entgegengesetzte Richtungen weisen. Dadurch wird erreicht, daß sich die beiden Strömungen nicht überlagern und Verwirbelungen im Bereich des Strömungsbauteils vermieden werden.

Um die Herstellung des Strömungsleitbauteils weiter zu vereinfachen, besteht dieses vorteilhafterweise aus zwei Teilstücken. Das erste Teilstück umfaßt vorzugsweise das zentrale Strömungsrohr und die obere Hälfte der Strömungsleitrohre und das zweite Teilstück die untere Hälfte der Strömungsleitrohre. Alternativ ist es aber auch möglich, das Strömungsbauteil aus zwei Längshälften zusammenzusetzen. Die beiden Teilstücke lassen sich im Spritzgußverfahren in entsprechenden Formen besonders einfach fertigen. Später können die Teilstücke unter Ausbildung der Strömungsleitkanäle zusammengesetzt werden, in denen die Flüssigkeit umgelenkt wird. Hierzu ist die eine Hälfte des Strömungsleitbauteils zweckmäßigerweise mit Stiften versehen, die beim Zusammendrücken der beiden Teilstücke in entsprechende Ausnehmungen der anderen Hälfte greifen. Die Teilstücke können auch miteinander verklebt werden.

Nachfolgend wird unter Bezugnahme auf die Zeichnungen ein Ausführungsbeispiel der erfindungsgemäßen Vorrichtung zum Abscheiden von Luftblasen aus medizinischen Flüssigkeiten beschrieben.

Es zeigen:
- Fig. 1: den Luftabscheider in teilweise geschnittener Darstellung,
- Fig. 2: das Strömungsleitbauteil des Luftabscheiders in der Seitenansicht,
- Fig. 3: das obere und untere Teilstück des Strömungsbauteils in der Seitenansicht vor dem Zusammensetzen,
- Fig. 4: eine Ansicht des unteren Teilstücks des Strömungsbauteils aus der Richtung des Pfeils IV von Fig. 3,
- Fig. 5: das untere Teilstück des Strömungsleitbauteils von Fig. 4 in der Draufsicht, und
- Fig. 6: das aus zwei Längshälften zusammengesetzte Strömungsleitbauteil in teilweise geschnittener, perspektivischer Darstellung.

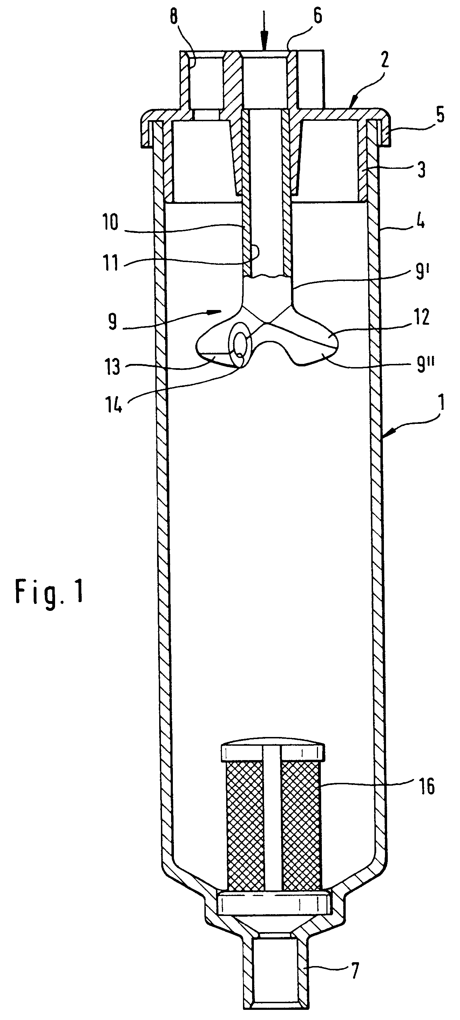

Fig. 1 zeigt den senkrecht angeordneten Luftabscheider in teilweise geschnittener Darstellung. Der Luftabscheider weist eine im wesentlichen kreiszylinderförmige Kammer 1 auf, die an ihrem oberen Ende mit einem Deckel 2 verschlossen ist, der mit einem zylindrischen Flansch 3 und einem umlaufenden, die Wand 4 des Behälters 1 übergreifenden Rand 5 versehen ist.

Im Zentrum des Deckels 2 ist ein Einlaufstutzen 6 zum Anschluß einer nicht dargestellten Zuführleitung und am unteren Ende des Behälters ist ein Auslaufstutzen 7 zum Anschluß einer nicht dargestellten Ablaufleitung angeordnet, die jeweils einen Innendurchmesser aufweisen, der zusammen mit dem Durchmesser der einzufügenden Anschlußschläuche eine Preßpassung bilden. Der Einlaufstutzen 6 ist einstückig mit dem Deckel 2 ausgebildet. Neben dem Einlaufstutzen 6 befindet sich am Behälterdeckel 2 ein weiterer Stutzen 8 zum Anschluß eines Druckschlauches.

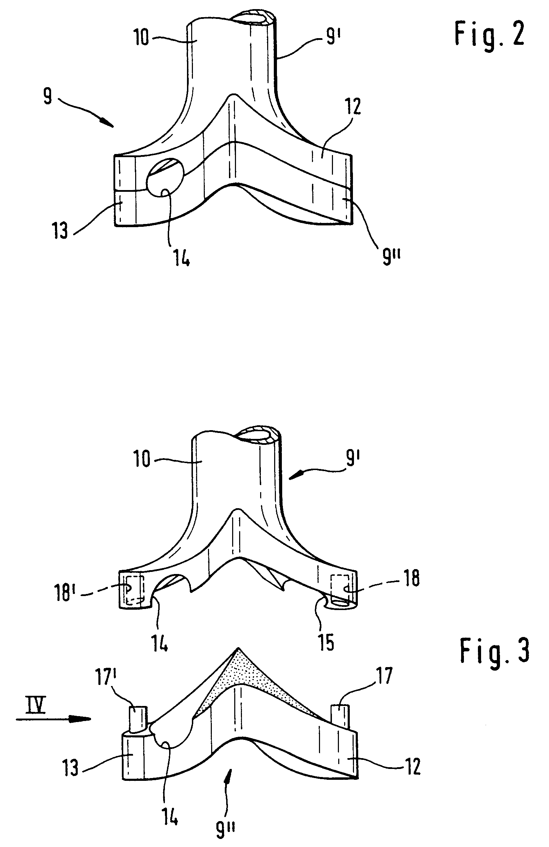

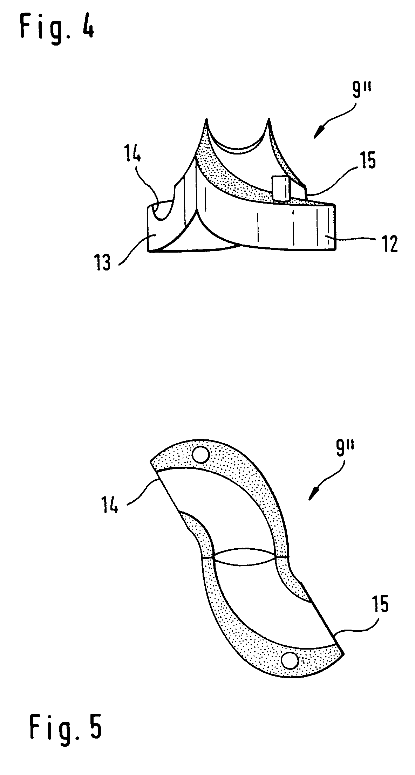

Stromabwärts des Einlaufstutzens 6 ist ein Strömungsleitbauteil 9 angeordnet, das im folgenden unter Bezugnahme auf die Fig. 2 bis 5 im einzelnen beschrieben wird.

Das Strömungsleitbauteil 9 weist ein zentrales, in Längsrichtung der Kammer angeordnetes Strömungsrohr 10 mit einem zentralen Strömungskanal 11 auf, das passend in den Einlaufstutzen 6 des Behälterdeckels 2 eingesetzt ist. Das zentrale Strömungsrohr 10 geht in zwei Strömungsleitrohre 12, 13 über, die einstückiger Bestandteil des zentralen Strömungsrohrs 10 und bezüglich der Längsachse des Strömungsrohres symmetrisch ausgebildet sind. Zur Verdeutlichung zeigt Fig. 1 das obere Teilstück des zentralen Strömungsrohres 10 in geschnittener Darstellung, während das untere Teilstück des zentralen Strömungsrohres 10 und die beiden Strömungsleitrohre 12, 13, d.h. der untere Teil des Strömungsleitbauteils 9, in perspektivischer Darstellung dargestellt sind. Die Strömungsleitrohre 12, 13 erstrecken sich ausgehend von dem in Längsrichtung der Kammer angeordneten zentralen Strömungsrohr 10 nach beiden Seiten in einer Raumkurve schraubenförmig in eine im wesentlichen tangential zur Wandung der Kammer 1 verlaufende horizontale Richtung. Die Öffnungen 14, 15 der beiden flügelartig abstehenden Strömungsleitrohre 12, 13 weisen dabei in entgegengesetzte Richtungen. Die Strömungsleitrohre weisen über ihre Länge einen im wesentlichen gleichbleibenden Kanalquerschnitt auf, der etwa halb so groß wie der Querschnitt des zentralen Strömungskanals 11 ist.

Durch die besondere Ausbildung des Strömungsleitbauteils 9 wird die in Richtung des oberen Pfeils durch den Einlaufstutzen 6 in das zentrale Strömungsrohr 10 einströmende Flüssigkeit in den beiden Strömungsleitrohren 12, 13 radial nach außen geleitet und dabei zusätzlich so umgelenkt, daß die Flüssigkeit beim Austritt aus den Strömungsleitrohren in einer im wesentlichen tangential zur Wandung der Kammer verlaufenden, horizontalen Richtung strömt (Fig. 1). Die vertikale Strömung wird also in eine horizontale Zirkulationsströmung umgelenkt. Hierdurch wird eine schraubenlinienförmige Strömung induziert, wobei die kreisförmigen Anteile einen Druckunterschied aufbauen, der dazu führt, daß die Luftblasen in Richtung Längsachse gedrängt werden und aufgrund ihrer geringeren Dichte nach oben aufsteigen. Die aufgestiegenen Luftblasen bilden im oberen Teil der Kammer ein Luftpolster. Generell sind möglichst geringe Strömungsgeschwindigkeiten anzustreben, um eine erneute Zufuhr von Luft an der Oberfläche der Flüssigkeit zu vermeiden. Ferner sollten die Austrittsöffnungen 14, 15 des Strömungsbauteils 9 in ausreichendem Abstand zu dem Deckel 2 bzw. des sich unterhalb des Deckels ausbildenden Luftpolsters angeordnet sein. Nachdem das Blut von oben nach unten in schraubenlinienförmigen Bahnen durch die Kammer 1 geflossen ist, fließt es durch eine in Fig. 1 mit dem Bezugszeichen 16 bezeichnete Filterkerze und dann durch den Auslaufstutzen 7 zur weiteren Verwendung ab. Fig. 1 zeigt eine koaxiale Anordnung von Ein- und Auslaufstutzen 6, 7. Es ist aber auch möglich, daß nur der Einlaufstutzen 6 in Kammerlängsrichtung angeordnet ist und der Auslaufstutzen 7 in tangentialer Richtung an die Kammer angeschlossen ist.

Aus fertigungstechnischen Gründen besteht das Strömungsleitbauteil 9 aus zwei Teilstücken 9', 9'', die in Fig. 3 dargestellt sind. Das erste Teilstück 9' umfaßt das zentrale Strömungsrohr 10 und die obere Hälfte der beiden Strömungsleitrohre 12, 13, während das zweite Teilstück 9'' die untere Hälfte der beiden Strömungsleitrohre 12, 13 umfaßt. Damit sich die beiden Teilstücke 9', 9'' nach der Fertigung einrastend zusammensetzen lassen, sind an der unteren Hälfte zwei Stifte 17, 17' vorgesehen, die in entsprechende Ausnehmungen 18, 18' an dem oberen Teilstück greifen und beide Teile fest zusammenhalten.

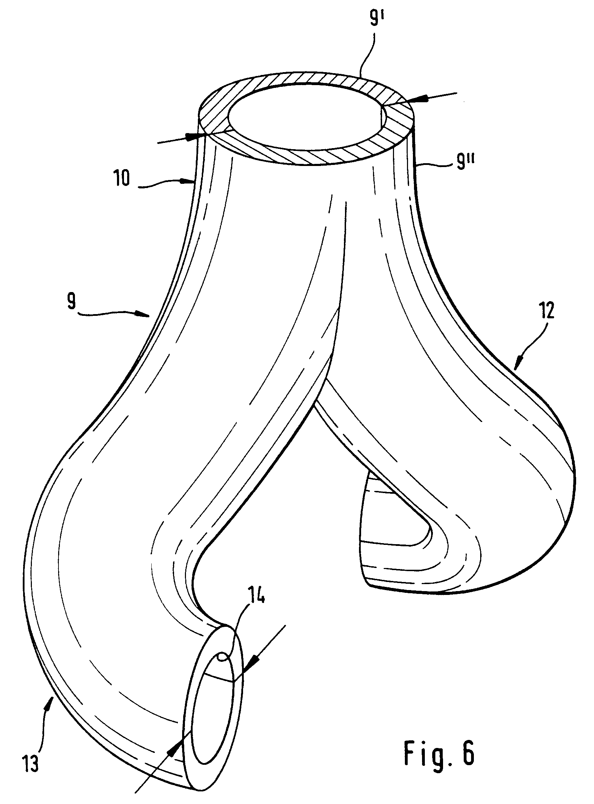

Fig. 6 zeigt das aus zwei Längshälften zusammengesetzte Strömungsleitbauteil in teilweise geschnittener perspektivischer Darstellung. Das Strömungsleitbauteil ist in Längsrichtung ohne Hinterschneidungen geteilt.

Dabei umfaßt das erste Teilstück 9' die eine Längshälfte des zentralen Strömungsrohres 10 und jeweils die eine Hälfte der Strömungsleitrohre 12, 13 und das zweite Teilstück 9'' umfaßt die andere Längshälfte des zentralen Strömungsrohres 10 und jeweils die andere Hälfte der Strömungsleitrohre 12, 13. Die Schnittlinie ist in Fig. 6 durch Pfeile gekennzeichnet. Bei dieser Ausführungsform weisen die beiden Teilstücke keine Stifte bzw. Ausnehmungen auf, sondern die beiden Teilstücke sind miteinander verklebt.

Sämtliche Teile des erfindungsgemäßen Luftabscheiders werden vorzugsweise im Spritzgußverfahren aus durchsichtigem Kunststoff hergestellt, so daß eine optische Kontrolle des Füllstandes und des Strömungsverlaufs jederzeit möglich ist.

Die erfindungsgemäße Ausbildung des Strömungsleitbauteils schafft einen Luftabscheider, der sich aufgrund einer möglichen koaxialen Anordnung von Einlauf- und Auslaufstutzen auf einfache Weise in ein bestehendes Schlauchsystem einfügen läßt und erlaubt eine Fertigung in großen Stückzahlen mit nur geringem technischen Aufwand.

## Patentansprüche

1. Vorrichtung zum Abscheiden Von Luftblasen aus medizinischen Flüssigkeiten, insbesondere Blut, mit einer im wesentlichen kreiszylinderförmigen Kammer (1), einem Einlaufstutzen (6) und einem Auslaufstutzen (7),
**dadurch gekennzeichnet,**
daß der Einlaufstutzen (6) in Längsrichtung der Kammer (1) angeordnet ist, und daß an den Einlaufstutzen (6) ein Strömungsleitbauteil (9) mit einem in Längsrichtung der Kammer (1) verlaufenden zentralen Strömungsrohr (10) angeschlossen ist, das in mindestens zwei Strömungsleitrohre (12, 13) übergeht, die sich als zylindrische Hohlkörper jeweils in einer kontinuierlichen Raumkurve aus der Kammerlängsrichtung in eine im wesentlichen tangential zur Wandung der Kammer verlaufende Richtung erstrecken.

2. Vorrichtung nach Anspruch 1, **dadurch gekennzeichnet**, daß das Strömungsleitbauteil (9) zwei Strömungsleitrohre (12, 13) aufweist, deren Öffnungen (14, 15) in entgegengesetzte Richtungen zeigen.

3. Vorrichtung nach Anspruch 2, **dadurch gekennzeichnet**, daß das erste und zweite Strömungsleitrohr (12, 13) bezüglich der Längsachse des zentralen Strömungsrohres (10) symmetrisch sind.

4. Vorrichtung nach einem der Ansprüche 1 bis 3, **dadurch gekennzeichnet**, daß das Strömungsleitbauteil (9) aus mindestens zwei Teilstücken (9', 9'') besteht.

5. Vorrichtung nach Anspruch 4, **dadurch gekennzeichnet**, daß das erste Teilstück (9') das zentrale Strömungsrohr (10) und die obere Hälfte der Strömungsleitrohre (12, 13) und das zweite Teilstück (9'') die untere Hälfte der Strömungsleitrohre (12, 13) umfaßt.

6. Vorrichtung nach Anspruch 4, **dadurch gekennzeichnet**, daß das erste Teilstück (9') die eine Hälfte des zentralen Strömungsrohres (10) und jeweils die eine Hälfte der Strömungsleitrohre (12, 13) und das zweite Teilstück (9'') die andere Hälfte des zentralen Strömungsrohres (10) und jeweils die andere Hälfte der Strömungsleitrohre (12, 13) umfaßt.

7. Vorrichtung nach einem der Ansprüche 4 bis 6, **dadurch gekennzeichnet**, daß die beiden Teilstücke (9', 9'') des Strömungsleitbauteils (9) zusammensetzbar sind.

8. Vorrichtung nach Anspruch 7, **dadurch gekennzeichnet**, daß ein Teilstück (9', 9'') des Strömungsbauteils (9) mit Stiften (17, 17') versehen ist, die in entsprechenden Ausnehmungen (18, 18') des anderen Teilstücks (9', 9'') sitzen.

9. Vorrichtung nach einem der Ansprüche 1 bis 8, **dadurch gekennzeichnet**, daß die Kammer (1) im wesentlichen senkrecht angeordnet ist.

## Claims

1. An apparatus for separating air bubbles from medical fluids, particularly blood, with a substantially circularly cylindrical chamber (1), an inlet union (6) and an outlet union (7), characterised in that the inlet union (6) is disposed in the longitudinal direction of the chamber (1) and in that connected to the inlet union (6) is a flow guiding component (9) with a central flow tube (10) extending in the longitudinal direction of the chamber (1) and which merges into at least two flow guiding tubes (12, 13) which extend as hollow cylindrical bodies respectively in a continuous spatial curve out of the longitudinal direction of the chamber into a direction extending substantially tangentially to the walls of the chamber.

2. An apparatus according to claim 1, characterised in that the flow guiding component (9) comprises two flow guiding tubes (12, 13) of which the orifices (14, 15) point in opposite directions.

3. An apparatus according to claim 2, characterised in that the first and second flow guiding tubes (12, 13) are symmetrical with reference to the longitudinal axis of the central flow tube (10).

4. An apparatus according to one of claims 1 to 3, characterised in that the flow guiding component (9) consists of at least two parts (9', 9'').

5. An apparatus according to claim 4, characterised in that the first part (9') comprises the central flow tube (10) and the upper half of the flow guiding tubes (12, 13) while the second part (9'') comprises the bottom half of the flow guiding tubes (12, 13).

6. An apparatus according to claim 4, characterised in that the first part (9') comprises one half of the central flow tube (10) and respectively one half of the flow guiding tubes (12, 13) and the second part (9'') comprises the other half of the central flow tube (10) and respectively the other half of the flow guiding tubes (12, 13).

7. An apparatus according to one of claims 4 to 6, characterised in that the two parts (9', 9'') of the flow guiding component (9) can be fitted together.

8. An apparatus according to claim 7, characterised in that one part (9', 9'') of the flow component (9) is provided with pegs (17, 17') which fit in corresponding recesses (18, 18') of the other part (9', 9'').

9. An apparatus according to one of claims 1 to 8, characterised in that the chamber (1) is disposed substantially vertically.

## Revendications

1. Dispositif pour éliminer les bulles de gaz d'un liquide médical, en particulier du sang, avec une chambre (1) de forme sensiblement cylindrique, une tubulure d'entrée (6) et une tubulure de sortie (7), caractérisé en ce que la tubulure d'entrée (6) est disposée en direction longitudinale de la chambre (1) et en ce que sur la tubulure d'entrée (6) est raccordé un composant de guidage du flux (9) avec un tube d'écoulement central (10) placé en direction longitudinale de la chambre (1), le composant de guidage du flux bifurquant en au moins deux tubes de guidage du flux (12, 13) qui s'étendent en tant que corps creux cylindriques respectivement selon une courbe spatiale continue à partir de la direction longitudinale de la chambre dans une direction sensiblement tangentielle à la paroi de la chambre.

2. Dispositif selon la revendication 1, caractérisé en ce que le composant de guidage du flux (9) comprend deux tubes de guidage du flux (12, 13) dont les ouvertures (14, 15) sont positionnées dans des directions opposées.

3. Dispositif selon la revendication 2, caractérisé en ce que les premier et deuxième tubes de guidage du flux (12, 13) sont symétriques par rapport à l'axe longitudinal du tube d'écoulement central (10).

4. Dispositif selon l'une des revendications 1 à 3, caractérisé en ce que le composant de guidage du flux (9) se compose au moins de deux pièces distinctes (9', 9'').

5. Dispositif selon la revendication 4, caractérisé en ce que la première pièce distincte (9') comprend le tube d'écoulement central (10) et la moitié supérieure des tubes de guidage du flux (12, 13) et que la deuxième pièce distincte (9'') comprend la moitié inférieure des tubes de guidage du flux (12, 13).

6. Dispositif selon la revendication 4, caractérisé en ce que la première pièce distincte (9') comprend une moitié du tube d'écoulement central (10) et respectivement l'une des moitiés des tubes de guidage du flux (12, 13) et que la deuxième pièce distincte (9'') comprend l'autre moitié du tube d'écoulement central (10) et respectivement l'autre des moitiés des tubes de guidage du flux (12, 13).

7. Dispositif selon l'une des revendications 4 à 6, caractérisé en ce que les deux pièces distinctes (9', 9'') du composant de guidage du flux (9) peuvent être assemblées.

8. Dispositif selon la revendication 7, caractérisé en ce qu'une pièce distincte (9', 9'') du composant de guidage du flux (9) est munie de broches (17, 17') qui pénètrent dans des évidements correspondants (18, 18') de l'autre pièce distincte (9', 9'').

9. Dispositif selon l'une des revendications 1 à 8, caractérisé en ce que la chambre (1) est disposée sensiblement à la verticale.
